Europäisches Patentamt

European Patent Office

Office européen des brevets

(19)

(11) Publication number: **0 386 960**
**A2**

(12) # EUROPEAN PATENT APPLICATION

(21) Application number: 90302256.4

(22) Date of filing: 02.03.90

(51) Int. Cl.5: **A61K 47/34, A61K 47/38,**
**A61L 15/28, A61K 9/06**

(30) Priority: 07.03.89 GB 8905138
28.09.89 GB 8921944

(43) Date of publication of application:
**12.09.90 Bulletin 90/37**

(84) Designated Contracting States:
**AT BE CH DE DK ES FR GB GR IT LI LU NL SE**

(71) Applicant: **AMERICAN CYANAMID COMPANY**
**Berdan Avenue**
**Wayne New Jersey 06904(US)**

(72) Inventor: **Gibson, Mark**
**18 Trueway Drive**
**Shepshed, Leicester LE12 9DU(GB)**
Inventor: **Taylor, Peter Mark**
**"The Loft", 3 Grove Road**
**Fareham, Hampshire PO16 7TF(GB)**
Inventor: **Payne, Nicholas Ian**
**50 Romsey Avenue**
**Fareham, Hampshire PO16 8TA(GB)**
Inventor: **Gould, Philip Leon**
**Rowan House, Mill Lane, Mickelton**
**Barnard Castle, Co. Durham DL12 0RG(GB)**

(74) Representative: **Allam, Peter Clerk et al**
**LLOYD WISE, TREGEAR & CO. Norman**
**House 105-109 Strand**
**London WC2R 0AE(GB)**

(54) **Pharmaceutical compositions useful as drug delivery vehicles and/or as wound dressings.**

(57) There is provided a pharmaceutical composition which comprises an aqueous vehicle, a compound having reversible thermosetting gel properties and a compound having film-forming properties. Depending on the relative proportions of these components, the composition can be adapted to function either as a vehicle for delivering pharmacologically or diagnostically active compounds to a human or animal patient and/or as a wound dressing composition.

EP 0 386 960 A2

# PHARMACEUTICAL COMPOSITIONS USEFUL AS DRUG DELIVERY VEHICLES AND/OR AS WOUND DRESSINGS

This invention relates to pharmaceutical compositions which are useful either as drug delivery vehicles, for delivering pharmacologically or diagnostically active compounds to a human or animal patient by means of enteral or parenteral routes, and/or as wound dressing compositions which can be adapted for application to superficial and deep-seated wounds.

As will become apparent from the description which follows, the compositions of this invention can be suitably adapted to serve either as a drug delivery vehicle or as a wound dressing (or in some cases as a wound dressing which simultaneously delivers a drug to the wound site). Nonetheless it is convenient to describe the delivery vehicle and wound dressing aspects of this invention separately.

## Drug Delivery Compositions

Conventional drug delivery compositions which are in the form of liquids, ointments or gels are not without problems, at least for certain applications.

For example, conventional liquid ophthalmic formulations suffer the disadvantage that most of the dose is lost, due to constant lacrimal (tear) secretion and nasolacrimal drainage, within the first 15-30 seconds post-instillation. In order to compensate for this loss, the patient may be subjected to frequent instillation of concentrated medication to achieve the desired therapeutic effect, but this practice in turn can result in undesirable side-effects because of systemic absorption of drug via the nasolacrimal duct. Recently, sustained release ophthalmic formulations using viscous gels or ointments, to prolong the corneal contact time of medication, have been proposed to overcome this problem (see e.g. Int. J. Pharm. 12 (1982), 147-152 and J. Pharm. Pharmacol. 41 (1989), 197-200). However, the ease of administration of gels and ointments, and the achievement of a measured dose, are much more difficult than for a liquid, and these factors can result in reduced patient compliance.

Conventional gels and ointments for topical delivery, particularly to the vagina and rectum, suffer the disadvantages that they are difficult to apply in this form and to obtain intimate contact with the contours of mucous membranes. Liquid formulations may be used to provide better initial contact, but they suffer the disadvantage that they do not remain localised in the specific region sufficiently long for drug absorption to take place. Further, all these conventional formulations may not always provide sufficient adherence to the tissue at the site of application to achieve adequate therapeutic effect.

Conventional viscous formulations for injection providing a depot in the tissue for sustained release, can be very painful when injected, and physically difficult to inject into the muscle tissue. In contrast, less viscous injections are easier to inject but may be quickly absorbed from the tissues so that no sustained therapeutic effect is achieved.

One potential problem with oral dosage forms for ingestion can be a narrow absorption "window" i.e. the area of the gastro-intestinal tract through which a given drug is absorbed. This often means frequent dosing of drugs to maintain effective therapeutic blood levels. This in turn may introduce patient compliance problems as well as variable blood drug concentrations.

The present invention aims to provide a novel drug delivery composition which imparts advantages which are not possessed by many conventional drug delivery systems.

In accordance with the present invention there is provided a drug delivery composition comprising an aqueous vehicle containing (a) from 1% to 30% by weight of one or more compounds having reversible thermosetting gel properties and (b) from 1% to 15% by weight of one or more compounds, compatible with component (a), having film-forming properties, and the composition having a sol-gel transition temperature within the range 0° to 50°C.

The drug delivery compositions of this invention will normally also contain from 0.001% to 35% by weight of at least one compound having pharmacological or diagnostic activity. However, in some instances, where the stability of the active compound is poor for example, it may be desirable to incorporate the active compound into the composition only just prior to application.

Depending on the sol-gel transition temperature, within the range of 0° to 50°C, and the temperature at which the composition is administered, the drug delivery compositions of this invention may be administered to a patient:

    (a) as a pourable liquid, which remains as a liquid at body temperature,
    (b) as a gel, which remains as a gel at body temperature, or

(c) as a pourable liquid which sets at body temperature to a gel, or

(d) as a gel which reverts to a liquid at body temperature.

In the preferred embodiments of the present invention, the compositions of this invention have a sol-gel transition temperature within the range 10° to 35°C, more preferably 15° to 30°C, whereby the compositions are semi-viscous pourable liquids at or about room temperature but set to form a gel in contact with the body of the human or animal patient. Such compositions may therefore be applied from a suitable container, which in many cases preferably will have been kept refrigerated (about 3° to 8°C), or otherwise maintained below their sol-gel transition temperature, at least immediately prior to use, by being poured onto or instilled or injected into the patient's body in order to deliver the pharmacologically or diagnostically active ingredient(s) to the required site. To ensure satisfactory fluidity at the time of use it is preferred that the viscosity of the aqueous compositions should be from 0.5 to 250 poise (Brookfield Model LV viscometer, No. 4 spindle) immediately below their sol-gel transition temperatures.

The compositions of this preferred embodiment of the invention can be used to administer a very wide variety of pharmacologically and diagnostically active substances by enteral or parenteral routes.

In general, the sol-gel transition temperature of these preferred compositions should be selected, within the range 10° to 40°C, having regard to the intended route of administration and the body site to which the active component is to be delivered. However, the transition temperature should not be below 10°C, since at a lower transition temperature there is a risk that the composition will set too quickly at ambient temperature, thus making it difficult, if not impossible, to administer before it has set. The maximum sol-gel transition temperature is set at 40°C, in the light of the fact that blood temperature is approximately 37°C for humans and most warm-blooded animals.

Thus, for instance, for topical application to the body tissues it is preferred that the sol-gel transition temperature should be between 10°-25°C, more preferably 15°-20°C. With a transition temperature above 25°C, the composition can take an undesirably long time to gel post-application, or in some instances may not gel at all. On the other hand, when used for ophthalmic purposes, the sol-gel transition temperature should desirably lie between 15°-35°C, and more preferably from 20°-26°C. With a transition temperature below 15°C, application to the eye can cause thermal shock and discomfort, whilst with a transition temperature above 35°C there is the risk of the product not gelling. For other routes of delivery, in contrast, any transition temperature within the permitted range generally can be used, depending upon the route of application.

After enteral or parenteral administration to the patient the composition sets to form what we term "a gelatinous film" or "gel-film" as its temperature is raised to that of the site to which it has been applied.

However, the characteristics of this gel-film vary somewhat, depending on the quantities of gel-forming and film-forming ingredients, the concentrations and nature of the pharmacologically active component and the possible presence of other ingredients. Indeed, it is a particular advantage of the present invention that by varying the amounts of the ingredients a range of different formulations can be provided to more clearly suit the requirements of different administration sites.

For instance, it is possible to formulate the composition so that, on setting, it forms a rigid gel which is particularly suitable for topical drug delivery to the skin or, for example, for the treatment of mouth ulcers i.e. in those circumstances where it is desired that the composition after administration should have intimate contact with the site of application and firmly adhere to it. Such a gel is non-flowable; for instance, the meniscus of the product will not move when it is tilted through 90°. It typically has the consistency of a firm "jelly" such as that produced when gelatin in water is allowed to set. More particularly, compositions which set to form such a rigid gelatinous mass can be formed from mixtures comprising from 3% to 30%, preferably 5% to 15%, by weight of the gel-forming component (a) and from 3% to 15%, preferably 5% to 10%, by weight of the film-forming component (b).

An alternative approach, employing lower amounts of gel-forming and film-forming components, provides a composition which sets to form a soft gel, and is particularly suitable, for instance, as a vehicle for ophthalmic drug delivery or in other circumstances where it is desired that the composition after administration should not cause disturbances of vision or excessive lacrimal (tear) formation, but still releases the drug over a reasonable period of time. By the term "soft gel" we mean a gel which is deformable under light pressure eg contact with a human finger. Typically, it has a viscosity of 50 to 2000 poise (Brookfield viscometer, Model LV, Spindle No. 4) at 25°C, eg a consistency of petroleum jelly. More particularly, compositions which set to form soft-gels can be formed from mixtures comprising from 1% to 10%, preferably 4% to 7%, by weight of the gel-forming component (a) and from 1% to 10%, preferably 4% to 7%, by weight of the film-forming component (b). Compositions which set to form soft-gels may also be formed from mixtures comprising from 1% to 5%, preferably 2% to 3%, by weight of the gel-forming component (a) and from 5% to 15%, preferably 8% to 12%, by weight of the film-forming component (b).

3

These latter compositions form soft gels with good film properties, ie upon application to the body they can form a tough, coherent film. This type of composition may also be suitable for topical delivery to the skin or superficial wounds, or for buccal delivery.

In all these compositions, the film-forming component (b) generally enhances the rate of gelation of the composition, controls the sol-gel transition temperature and, in some instances may actually increase the gel strength of the set composition.

Of course, it will be understood that the compositions of this invention may be formulated so as to have properties intermediate between those of the two types of formulation just referred to.

Although, as described above, the preferred compositions of this invention have sol-gel transition temperatures such that they can be applied as pourable liquids which then set, post-administration, to form a gel, there may nonetheless be circumstances in which different modes of administration and/or different properties post-administration are required.

For example, if it is desired to administer the composition as a pourable liquid which remains as a liquid at body temperature, the composition is formulated so that it has a high sol-gel transition temperature, eg in the range 40°C to 50°C. In contrast to many conventional liquid drug delivery systems, the compositions of this embodiment of the invention will have enhanced bioadhesive properties and hence maintain intimate contact with mucous membranes for longer periods of time. Moreover, the compounds which we prefer to use as the gel-forming component (as discussed below), possess surfactant properties, and accordingly, the present compositions will more readily dissolve poorly water-soluble drugs than conventional aqueous vehicles.

Another possibility is to formulate the composition so that it is administered as a gel and remains as a gel at body temperature. For this purpose, the sol-gel transition temperature of the composition should lie within the range of 0° to 15°C, so that when applied at ambient temperature the composition will already be in gel form. Such a pre-gelled composition could, for example, be advantageous for delivering a drug for treatment of a mouth ulcer whereas the pourable liquid compositions in accordance with this invention would be difficult to maintain at the site of application sufficiently long to set to a gel.

In yet other cases, it may be desirable to administer the composition as a gel which then forms a solution at body temperature. In these cases, the composition should be formulated so as to have a sol-gel transition temperature in the range 35° to 40°C, ie just around normal body temperatures for humans and warm-blooded animals. Administration of the compositions as a gel can be achieved by first warming the composition to a temperature just above its gel-sol transition eg to just above 40°C, so that the composition sets to a gel which will then revert back to a liquid post-administration at body temperatures. Thus, such a composition could be administered as a pessary or suppository in gel form which upon cooling will form a liquid to enhance the release of the drug.

Amongst the factors which can be varied in order to control the sol-gel transition temperature of the present compositions to within a desired temperature range may be mentioned the absolute and relative concentrations of the gel-forming and film-forming ingredients, the nature and amounts of additional ingredients, e.g. pharmacologically or diagnostically active compounds or other optional ingredients such as those to be discussed below.

A wide range of compounds may be used to provide the gel-forming and film-forming ingredients, respectively, of our compositions.

As is known to those skilled in the art, a number of compounds are available which exhibit reversible gelation properties and which are non-toxic and non-antigenic, as is required for pharmaceutical purposes. Such gel-forming materials are generally block copolymers of polyoxyethylene-polyoxypropylene, for instance those available under the trade names "Pluronic" and "Synperonic"; or tetrasubstituted derivatives of ethylene diamine where the substituents are block copolymers of polyoxyethylene-polyoxypropylene, for instance those available under the trade names "Synperonic T" and "Tetronic" (see, for example, GB-A-1571832 and EP-A-0126684). It is preferred that the gel-former should exhibit reversible thermal gelation properties at or around skin or body temperatures, ie within the range from 10°-45°C. We have found that Pluronic F127 is particularly suitable for the purposes of providing the gel-former in the compositions of this invention. This compound has an extremely low order of toxicity without adverse effects on mucous membranes. Pluronic F127 is a polyoxyethylene-polyoxypropylene block copolymer of average molecular weight about 11500.

A mixture of two or more different gel-formers may be used if desired.

Similarly, a number of different film-forming compounds known in the art can be used herein. Film-forming compounds suitable for use herein are compounds which are soluble in water and whose aqueous solutions dry to form a self-supporting film. Additionally, the film-forming compound must, of course, be compatible with the selected gel-former and preferably should be miscible therewith to avoid phase

4

separation. Further, the film-forming compounds should be non-toxic and non-antigenic. Moreover, the film-forming compound desirably should enhance the gel strength and be able to modify the sol-gel transition temperature, or gel setting time of the composition.

We currently prefer to use hydroxyethylcellulose, hydroxypropylmethylcellulose or polyvinyl alcohol as the film-forming ingredient. Low viscosity grade hydroxyethylcellulose, such as that sold by Hercules, Inc., UK under the trade mark "Natrosol 250 L" and with a viscosity (5% aqueous solution at 25°C, Brookfield LV viscometer with No. 4 spindle) in the range 100 to 180 centipoise, is especially advantageous since not only can it be incorporated in relatively high concentrations, for good film-forming properties, without unduly increasing the viscosity of the compositions at the time of application, but more particularly because it is highly compatible and miscible with Pluronic F127, our preferred gel-forming compound, and actually serves to enhance the gel strength of this gel-forming compound above the sol-gel transition temperatures. Thus, whereas, for instance, the viscosity of Pluronic F127 (10% w/w) in water is typically 0.2 poise, and the viscosity of Natrosol 250 L (10% w/w) in water is typically 2.3 poise, that of a mixture of Pluronic F127 and Natrosol 250 L (each at 10% w/w) in water is typically 9500 poise, producing a very strong and rigid gel. (These viscosity measurements were all made at 25°C using a Brookfield viscometer, Model LV, No. 4 spindle.) These results are highly surprising since, as is taught in US-A-4474753, for instance, it has previously been considered that the ability to obtain a gel of desired rigidity at physiologically useful sol-gel transition temperatures using commercially available Pluronic-type polymers has been limited.

Again mixtures of film-forming compounds can be used if desired, or for the purposes of obtaining specific properties.

Any pharmacologically or diagnostically active compound, or mixtures of such compounds, can be incorporated with the gel-forming and film-forming components, either initially when the drug delivery composition is manufactured or if necessary just prior to administration to a patient. Preferably, the active component will be soluble in the aqueous vehicle, to facilitate manufacture and to avoid sedimentation and caking problems, but insoluble compounds can be suspended in the vehicle by thorough mixing of the compounds with the other components of the composition by art-recognised techniques, care then being taken that the particle size of the suspended insoluble drug is appropriate (for instance a particle size of less than 10 μm is needed for ophthalmic or injectable compositions). The amount of the pharmacologically or diagnostically active component should be from 0.001 to 35% by weight, depending on such factors as its effect on the sol-gel transition temperature and gel viscosity (in the case of water-soluble compounds), the relative potency of the drug and therapeutic concentration required, the possibility of drug-related side effects and the ease of drug absorption at the site of application.

A very wide variety of different pharmacologically or diagnostically active compounds may be incorporated in the compositions of this invention, singly or in appropriate cases, in admixture, the following being exemplary but non-limiting.

## For Ophthalmic Drug Delivery

Steroidal anti-inflammatory drugs such as clobetasone butyrate, cortisone, hydrocortisone, hydrocortisone acetate, betamethasone, betamethasone sodium phosphate, dexamethasone, prednisone, methyl prednisolone, medrysone, fluorometholone, fluocortolone, prednisolone, prednisolone sodium phosphate, prednisolone acetate and triamcinolone. Non-steroidal anti-inflammatory drugs and their salts such as biphenylacetic acid, indomethacin, ketoprofen, ibuprofen, sulindac, piroxicam, fenoprofen, flurbiprofen, naproxen, diclofenac and oxyphenbutazone.

Antihistamines and decongestants such as antazoline sulphate, antazoline phosphate and chlorpheniramine.

Antibacterial compounds such as beta-lactam antibiotics (carbenicillin, penicillin G, cefoxitin), imipenem and other derivatives of thienamycin, chloramphenicol, tetracyclines such as chlortetracycline hydrochloride and tetracycline hydrochloride, aminoglycoside antibiotics such as gentamicin (sulphate) framycetin (sulphate), neomycin (sulphate), gramicidin, kanamycin, amikacin, sissomicin and tobramycin. Sulphonamides such as sulphacetamide sodium. Colistin and its salts, polymyxin B, vancomycin. Cephalosporin antibiotics such as cephazolin, cephalothin sodium, cephaloridine and the like. Nalidixic acid, norfloxacin and analogues of nalidixic acid.

Miotics and anticholinergics such as bethanechol (chloride), carbachol, demecarium bromide, ecothiapate iodide, methacholine (chloride), di-isopropyl-fluorophosphate, neostigmine (and its salts), physostigmine (and its salts), pilocarpine (and its salts), pyridostigmine bromide. Sympathomimetics such as adrenaline and derivatives, dipivefrine hydrochloride, naphazoline (and its salts), xylometazoline hy-

5

drochloride, phenylephrine hydrochloride, tetrahydrozoline hydrochloride and the like.

Mydriatics such as atropine, homatropine, scopolamine, hydroxyamphetamine, cyclopentolate hydrochloride, eucatropine hydrochloride, oxyphenonium bromide, tropicamide and the like.

Other medicaments used in the treatment of eye conditions or diseases such as:

Anti-glaucoma drugs such as carbonic anhydrase inhibitors, for example, acetazolamide, dichlorphenamide, methazolamide and the like. Also, $\beta$-blockers such as timolol, R-timolol and its salts and combinations, eg with pilocarpine. Hyperosmotic agents such as glycerol, mannitol and urea.

Antifungal agents such as amphotericin B, nystatin, natamycin, miconazole and flucytosine.

Anti-viral compounds such as acyclovir, adenosine arabinoside (Ara-A), idoxuridine, trifluorothymidine, interferon and interferon inducing agents, eg Poly I:Poly C.

Anti-parasite agents and/or anti-protozoal compounds such as clindamycin and corticosteroid preparations, ivermectin, pyrimethamine and trisulphapyrimidine.

Anaesthetics such as amethocaine, benzocaine, bupivacaine, cocaine, lignocaine, dyclonine hydrochloride, oxybuprocaine, etidocaine, phenacaine hydrochloride, piperocaine hydrochloride, propanocaine hydrochloride, proxymetacaine, hexylcaine hydrochloride, mepivacaine and prilocaine.

Ophthalmic diagnostic agents such as those used to examine the retina, conjunctiva, cornea, lacrimal areas, or to examine abnormal pupillary responses such as fluorescein sodium and rose bengal stains, methacholine, cocaine, adrenaline, atropine and pilocarpine.

Ophthalmic agents used as adjuncts to surgery such as alpha-chymotrypsin and hyaluronidase.

Immunosuppresive agents and anti-metabolites such as azathioprine, methotrexate, cyclophosphamide.

Chelating agents such as EDTA and deferoxamine.

Combinations of the above, eg antibiotic anti-inflammatory.

For Parenteral Delivery by Injection

Analgesics/anti-inflammatory compounds such as aspirin, salicylates, diclofenac sodium, biphenylactic acid, phenylbutazone, indomethacin, ibuprofen, diflunisal, fenbufen, fenoprofen, ketoprofen, sulindac and other non-steroidal anti-inflammatory compounds. Corticosteroids such as dexamethasone and the like.

Anaesthetics such as lignocaine, procaine, benzocaine and the like.

Anti-asthma compounds such as beclomethasone, ephedrine, theophylline and the like.

Anti-coagulants such as heparin, warfarin, bishydroxycoumarin and the like.

Anti-convulsants such as diazepam, phenytoin and the like.

Anti-depressants and anxiolytics such as chlordiazepoxide, imipramine, amitriptyline, doxepin and the like.

Urinary tract antiinfectives such as trimethoprim, sulphamethoxazole, norfloxacin and the like.

Anti-diabetics such as insulin, tolbutamide, chlorpropamide, acetohexamide, tolazamide and the like.

Anti-tumour/Anti-neoplastics such as adriamycin, fluorouracil, methotrexate, trimelamol, mitoxantrone, bisantrene and the like.

Anti-hypertensives such as clonidine, chlorothiazide, timolol, propranolol, metoprolol, methyldopa, prazosin hydrochloride, spironolactone, hydralazine, bisoprolol, reserpine and the like.

Muscle relaxants such as dantrolene sodium, cyclobenzaprine hydrochloride, methocarbamol, pancuronium bromide, suxamethonium chloride and the like.

Anti-psychotics such as lithium (carbonate or citrate), fluphenazine, amitriptyline, thioridazone and the like.

Contrast agents: examples suitable for use in the present invention include, but are not limited to the following: N,N'-bis[2-hydroxyl 1-(hydroxymethyl)ethyl]-5-[(2-hydroxy-1-oxopropyl)-amino]-2,4,6-triiodo-1,3-benzenedicarboxamide (Bracco 15,000), metrizamide, diatrizoic acid, sodium diatrizoate, meglumine diatrizoate, acetrizoic acid and its soluble salts, iodipamide, sodium iodamide, meglumine iodamide, iodohippuric acid and its soluble salts, iodopyracetiodo-2-pyridone-N-acetic acid and its soluble salts, 3,5-diiodo-4-pyridone-N-acetic acid (iodopyracet), 3,5-diiodo-4-pyridone-N-acetic acid diethanolamine salt, iodo-2-pyridone-N-acetic acid and its amine salt, iothalamic acid and its soluble salts, metrizoic acid and its soluble salts, sodium ipodate, iopanoic acid, iocetamic acid, tyropanoate sodium, iopydol, iophendylate, and other chemically related iodinated contrast agents. Where applicable, the contrast agents which may be employed herein include inorganic and organic salts of the above contrast agent, such as the potassium salt, calcium salt, lithium salt, arginine salt, cysteine salt, glycine salt, glycyl-glycine salt, N-methyl glucosamine salt and other non-toxic aliphatic and alicyclic amines employed in preparing water soluble salts.

Proteins, peptides and hormones such as insulin, monoclonal antibodies or monoclonal antibodies linked to drugs such as anti-tumour compounds, and the like.

## For Topical Delivery to the Skin

Suitable drugs which can be administered by the present delivery system include:

Anti-inflammatory drugs (see ophthalmic list above), including topical steroids such as aclometasone dipropionate, fluocinolone acetonide, clobetasone butyrate, beclomethasone dipropionate, triamcinolone acetonide and the like, analgesics (see parenteral injection list above), anti-histamines such as mepyramine maleate, antazoline, diphenhydramine hydrochloride, promethazine, dibromopropamidine and the like. Anaesthetics (see ophthalmic list above). Anti-infectives such as iodine, povidone-iodine benzalkonium chloride, phenol, cetyl pyrridinium chloride, chlorhexidine and the like, anti-fungals such as benzoyldisulphide, zinc undecylenate, undecylenic acid, tolnaftate, nystatin, natamycin. Imidazoles such as clotrimazole, miconazole, sulconazole nitrate, econazole nitrate, ketoconazole and the like.

Anti-parasitic compounds such as ivermectin, benzyl benzoate, carbaryl, malathion, monosulfiram, lindane and the like. Anti-viral compounds such as idoxuridine, acyclovir and interferon.

Anti-bacterial compounds such as cefoxitin, imipenem and other derivatives of thienamycin, chlortetracycline, choramphenicol, neomycin, gramicidin, bacitracin, silver sulphadiazine, gentamicin, framycetin sulphate, kanamycin and tobramycin, nalidixic acid, norfloxacin, fusidic acid and its salts, mupirocin, polymyxin B sulphate and the like.

Emollients and anti-pruritics such as urea, glycerin, sodium pyrrolidone carboxylate and crotamiton.

Anti-histamines such as diphenhydramine and the like.

Anti-psoriasis compounds such as dithranol.

Anti-acne compounds and keratolytics such as the vitamin A derivatives isotretinoin and tretinoin, resorcinol, minocycline (hydrochloride), erythromycin, doxycycline, salicyclic acid and benzoyl peroxide.

Wart removers such as formaldehyde, glutaraldehyde, benzalkonium chloride bromine and the like.

Sunscreen compounds e.g. UV protectants such as amino-benzoic acid and the like.

Anti-perspirants such as aluminium chloride hexahydrate, aluminium chlorohydrate and the like.

Hair restoring compounds such as minoxidil.

Wound management compounds such as chemotactic agents, desloughing agents, exudate absorbents such as dextranomer and cadexomer-iodine and the like, fibrinolytic agents such as streptokinase-streptodornase (VaridaseTM).

Anti-neoplastic compounds such as methotrexate, adriamycin, 5-fluouracil and the like.

## For Vaginal Drug Delivery

Anti-infective compounds such as anti-fungal agents nystatin, natamycin, imidazoles such as econazole, isoconazole, clotrimazole, miconozole, fluconazole, metronidazole, ketoconazole and the like.

Sulphonamides such as sulphathiazole, sulphacetamide and the like.

Anti-virals such as acyclovir, inosine pranobex, interferon and podophyllotoxin. Antiseptics such as povidone-iodine, chlorhexidine and the like.

Drugs used to treat urinary tract infections such as quinoline compounds such as cinoxacin and nalidixic acid, alkalising agents such as sodium citrate, citric acid, nitrofurans such as nitrofurantoin, hexamine, penicillins such as procaine penicillin, ampicillin and the like, aminoglycoside antibiotics such as gentamicin and the like, cephalosporins and cephamycins, trimethoprim, tetracyclines, polymyxin B sulphate. Urinary analgesics and anti-inflammatory drugs such as aspirin and other NSAI compounds such as mefenamic acid, naproxen sodium and the like.

Anaesthetics such as lignocaine hydrochloride and the like.

Drugs acting on the smooth muscle to induce labour or abortion such as oxytocin, and prostaglandins, such as alprostadil, dinoprost, gemeprost, dinoprostone and the like.

Drugs used to prevent and treat haemorrhage such as ergometrine maleate and/or combined with oxytocin, anti-fibrinolytic drugs such as tranexamic acid, haemostatic drugs such as ethamsylate.

Myometrial relaxants such as ritodrine HCl, beta-agonists such as terbutaline sulphate, isoxsuprine HCl, salbutamol and the like.

Spermicidal contraceptives such as nonoxinols, oxtoxinols and the like.

For Rectal Drug Delivery

Drugs for local therapy or systemic effect can be administered.

Anti-infective compounds such as anti-fungal agents such as metronidazole and the like. Antibiotics such as penicillins, sulphonamides, tetracyclines and the like. Contrast agents such as barium salts and the like.

Anti-inflammatory/analgesics such as aspirin, paracetamol, non-steroidal anti-inflammatory drugs such as indomethacin, naproxen, biphenylacetic acid and the like, narcotic analgesics such as oxymorphone hydrochloride and the like. Corticosteroids such as hydrocortisone, prednisolone, methylprednisolone and the like.

Anaesthetics such as lignocaine and the like.

Anti-asthmatics such as the xanthines such as theophylline, aminophylline and the like.

Anti-convulsant drugs such as diazepam and anxiolytics, anti-psychotic drugs such as chlorpromazine and the like.

Anti-emetics such as cyclizine, domperidone, prochlorperazine and the like.

Anti-histamines such as promethazine and the like.

Barbiturates such as amylobarbitone sodium and the like.

Proteins and peptides such as insulin and the like.

Laxatives such as bisacodyl and the like. Anti-haemorrhoid compounds such as crotamiton and bismuth subgallate.

For Buccal and Sublingual Drug Delivery

Anti-anginal drugs such as nitrates such as glyceryltrinitrate, isorsorbide dinitrate and the like. Anti-hypertensives such as nifedipine.

Hormones such as methyl testosterone, testosterone, oxytocin, insulin, estradiol and the like.

Anaesthetics such as benzocaine, lignocaine and the like.

Analgesics such as aspirin, choline salicylate, buprenorphine hydrochloride, methadone HCl and the like. Nicotine as an anti-smoking agent.

Anti-histamines such as chlorpheniramine, prochlorperazine and the like.

Anti-depressants such as imipramine HCl and the like.

Barbiturates such as amylobarbitone sodium and the like.

Treatments for mouth ulcers containing combinations of anaesthetics, steroids such as hydrocortisone and the like, and anti-infectives such as minocycline, fusafungine and the like, and anti-inflammatories such as carbenoxolone sodium, and the like.

Anti-fungals such as miconazole, amphotericin B, antibacterials such as chlorhexidine, metronidazole, nimorazole, nystatin, clioquinol and the like.

For Nasal Drug Administration

Anti-histamines such as antazoline and the like, corticosteroids such as beclomethasone, flunisolide, betamethasone, dexamethasone and the like.

Antibiotics such as mupirocin, neomycin sulphate. Sympathomimetics such as oxymetazoline hydrochloride, tramazoline hydrochloride, phenylephrine hydrochloride, xylometazoline hydrochloride.

Systemic delivery of beta-blockers such as propranolol and the like. Proteins, peptides such as oxytocin and desmopressin, and hormones such as insulin and buserelin acetate. Vaccines.

For Oral Drug Delivery

Antacids, anti-spasmodics, laxatives, anti-diarrhoeals, anti-anginals, diuretics, anti-hypertensives, anti-coagulants, anti-thrombotics, fibrinolytics, haemostatics, hypolipidaemic agents, hypnotics, anxiolytics, anti-psychotics, anti-depressants, anti-emetics, anti-convulsants, CNS stimulants, analgesics, antipyretics, anti-inflammatories, muscle relaxants, neuromuscular drugs, hormones, hyper-and hypo-glycaemics, thyroid and anti-thyroid drugs, anti-infective compounds, such as anti-biotics, anti-bacterials, anti-fungals, anti-tuberculous and anti-leprotic drugs, anti-malarials, anthelmintics and amoebicides, anti-virals. Vaccines and immunosuppressants. Nutritional compounds such as vitamins, iron, electrolytes, anti-obesity drugs, anabolic drugs. Anti-asthma drugs, expectorants, mucolytics, decongestants and anti-tussives. Anti-allergic drugs. Oral contraceptives. Anti-neoplastic-anti-tumour drugs. Contrast agents such as barium salts and the like.

A feature of the preferred compositions of the present invention is that by controlling the viscosity of the gel which forms post-administration, the rate of release of the pharmacologically active component can be controlled to some extent. Thus, an increase in gel viscosity generally results in a decrease in the rate of drug release from the gel, whilst conversely a decrease in gel viscosity results in a more rapid rate of drug release. The gel viscosity can thus be varied with this factor in mind, by adjustment of the content and relative proportions of the gel-forming and film-forming components.

The aqueous vehicle will normally consist of water alone, although in some instances it may also comprise small amounts eg up to 20% w/w, of a miscible, non-toxic and non-antigenic organic liquid eg polyethylene glycol, for instance, in order to improve the conformability or improve the 'skin-feel' of a topical drug delivery system.

As indicated above, other ingredients may optionally be included in the drug delivery compositions of this invention in order to modify their properties. For instance, we have found that incorporating a hydroxy- or polyhydroxy- compound enhances the gel strength, lowers the sol-gel transition temperature, and increases the physical stability of the composition, thus reducing the possibility of phase separation occurring during storage. Suitable stabilizing compounds include benzyl alcohol, glycerol, propylene glycol, ethanolamine, mannitol and sorbitol. We currently prefer to incorporate sorbitol in amounts of from 0.5% to 20%, more preferably from 1% to 10% by weight of the composition.

Another optional ingredient is a preservative, to protect the composition from microbial contamination during manufacture, storage and usage, particularly if the product is required to be sterile, or if used for multi-dose purposes where the material is dispensed repeatedly from the same container, as would typically be the case for ophthalmic drug delivery. The choice of preservative used will depend on factors such as compatibility with the other components of the composition and the intended site of application to the patient's body eg skin, eyes, mouth etc. Suitably, the preservatives may be employed in amounts from 0.001% to 5% by weight of the composition, preferably 0.01% to 2%. Preferably the preservatives are water-soluble. Examples of useful preservatives include benzyl alcohol, methyl and propyl parabens, chlorbutanol, benzalkonium chloride, cetyl pyrridinium chloride, ascorbate, sodium thiosulfate, sodium bisulfite, thiomersal, phenylmercuric nitrate and 2-phenoxyethanol.

However, the use of a preservative may affect the sol-gel transition temperature and gel-viscosity, and this effect therefore needs to be taken into account in determining the amounts of the other ingredients used when that preservative is included.

In general, the pH of the present compositions can range from pH 3 to 9. Gelling temperature and strength may be affected to some extent by the pH of the composition, although we have found that a pH within the range of 4 to 8 generally has no significant effect on either gel strength or gelling temperature. In some cases there may be an optimum pH for the solubility, stability or pharmacological activity of the drug, and/or preservative if included, in the composition. If necessary, the pH of the compositions can be adjusted to achieve a desired level using any pharmaceutically acceptable acid or base. Sodium hydroxide or ethanolamine, for instance, may be used to raise the pH, whilst hydrochloric acid is suitable for reducing the pH. Buffering agents may, if desired, be employed in amounts sufficient to maintain the pH of the system at a desired level, for example alkali or alkaline earth phosphates, citrates, bicarbonates, carbonates, acetates, borates, succinates and the like are suitable water-soluble buffering agents useful herein.

In many cases it is necessary that the drug delivery compositions of this invention should be sterile at the time of administration. We have found that terminal methods of sterilization of the compositions contained in the final container, such as by gamma irradiation or heating in an autoclave, may cause an unacceptable loss in viscosity of the gelled product. Sterilization of the composition through a filter into a sterile container is therefore the preferred sterilization technique for those compositions containing relatively smaller amounts of the gel-forming and film-forming components, when the viscosity of the composition below its sol-gel temperature permits and it is possible to maintain the composition below the sol-gel transition temperature to complete the filtration.

If filter sterilization is not feasible due to high product viscosity, as is often the case for compositions containing large amounts of the gel-forming and film-forming components, then a combination of sterilization techniques may be necessary. For example filtration of the aqueous part of the vehicle and gamma-irradiation of other components may have to be employed and the product manufactured aseptically in a sterile area.

For example, compositions in accordance with the present invention may, in general, be prepared as follows: the pharmacologically active substance, along with various additives such as buffers, preservatives

9

and stabilizing agents are dissolved in water at room temperature with continuous agitation. The gel-forming component e.g. Pluronic F127, is slowly added to the solution with gentle mixing at room temperature until hydration and dissolution is completed. It may take from 1 to 2 hours to effect complete dissolution of the gel-forming component. Thereafter, the resulting solution is sterilized by filtration through a 0.22 $\mu$m filter and transferred to a previously sterilized mixing vessel in a sterile handling area. The film-forming component e.g. hydroxyethylcellulose, previously sterilized by -irradiation (e.g. 2.5 MRads), is now added to the solution in the mixing vessel under aseptic conditions.

Aliquots of the resulting solution are then filled into sterile containers suitable for the intended application at a temperature below the sol-gel transition temperature. For example, containers such as sachets, tube dispensers, propellant-type aerosol spray containers or mechanically operated spray dispensers may be used for compositions intended for topical delivery. A reclosable bottle or the like fitted with an eye-dropper is suitable for ophthalmic compositions, whilst a syringe will normally be used as the container for compositions to be delivered by injection.

The filled containers may, but need not be, stored at a temperature below the sol-gel transition temperature of the compositions. If, however, the compositions are subjected at some stage before use to a temperature above the sol-gel transition temperature then it will be necessary to maintain the container at a temperature below the transition temperature for a short while immediately before use, if the composition needs to be fluid at the time of its administration.

The drug delivery compositions of this invention have a number of advantages. In particular, the compositions can exhibit a sol-gel transition temperature and gel viscosity which can be controlled over a wide range by appropriate selection of the gel-forming and film-forming components, and amounts thereof, so as to render them particularly suitable for the intended application.

Moreover, the preferred compositions offer the practical advantage of forming a gelatinous-film in situ at the site of administration, being easy to apply with the minimum of trauma to the patient. Variation of the viscosity of the gel formed at the site of application can be used to control the rate of drug release from the formulation. The compositions possess bioadhesive and muco-adhesive properties which enables them to adhere to dry or moist tissue at the site of application and can be used to increase the time the drug is in contact with the tissue, thereby permitting reduced frequency of dosing and increased patient compliance.

As a vehicle for delivering a drug by intramuscular or subcutaneous injection, the present preferred compositions have the advantage that they can be injected as a relatively low viscosity liquid (ie below the sol-gel transition temperature) to reduce the pain which the patient experiences, but after injection they gel at body temperature to form a drug depot for sustained release.

In general, liquid pharmaceutical formulations are more convenient to handle during manufacture, filling into containers for storage, dispensing and administration to the patient. However, they usually provide a reduced therapeutic effect when compared to more viscous formulations, because the drug is more rapidly absorbed or dispersed at the site of application. The present compositions permit the formulation of drug delivery systems which are in the liquid state up to and including the time of administration, with the attendant advantages that such liquid formulations possess at those stages, but which after administration forms a semi-solid gel at the administration site to provide the enhanced therapeutic effect of viscous formulations.

## Wound Dressing Compositions

As previously indicated, this invention also provides a novel type of wound dressing which can be adapted for application to both superficial and deep-seated wounds.

In recent years the traditional woven and non-woven sheet-form wound dressings have been supplemented by a variety of newer types of wound dressing. Amongst these recently introduced wound dressings are so-called film dressings and gel dressings.

Film dressings consist of a transparent, semi-permeable film of polyurethane or other synthetic polymer, often coated on one side with an adhesive by which the dressing can be adhered to the wound site. They are now widely used, in the treatment of superficial wounds and burns, their permeability to air and water vapour allowing the tissue to remain in an environment conducive to optimum wound healing. However, film dressings do not absorb excess exudate and toxic components from the wound, which means that generally they are unsuitable for treating deep cavity wounds. Moreover, film dressings can be difficult to apply to certain areas, for example around fingers, toes and joints. Furthermore, they may allow infections to develop underneath the film as a result of tracking of bacteria along ridges and folds in the dressing if good adhesion to the wound has not been achieved.

Gel dressings are available either as self-supporting sheet-like products, as gels or in a non-gelled form capable of forming a gel in situ at the wound site. Unlike film dressings, gel dressings are useful for the treatment of cavity wounds. They provide a material which may expand or otherwise fill the wound cavity and once applied they provide a moist interface to potentiate wound healing. Moreover, gel dressings are often capable of absorbing excess exudate. However the presently available gel dressings all have the disadvantage of lacking mechanical strength, and consequently, and in contrast to film dressings, they usually require an additional dressing to hold them in place. Further, these gel dressings can be difficult to apply, and in some instances they leave residues which become implanted in new tissue growth.

The currently available film and gel wound dressings have been described in more detail in the literature, see for example Lawrence in "Pharmacy Update", April 1987, pages 147-150; and Turner in "Pharmacy International", June 1985, 131-134.

The object of the present invention is to provide a new wound dressing which has both film- and gel-forming properties, whereby the wound dressing has a combination of advantageous properties not possessed by the existing film and gel dressings.

In accordance with the present invention there is provided a wound dressing composition comprising an aqueous vehicle containing (a) from 1% to 30% by weight of the composition of one or more compounds having reversible thermosetting gel properties, and (b) from 1% to 20% by weight of the composition of one or more compounds, compatible with component (a) having film-forming properties, and the composition having a sol-gel transition temperature within the range 10° to 30°C, a viscosity of not greater than 250 poise, as determined by a Brookfield LV viscometer with No. 4 spindle, at a temperature immediately below its sol-gel transition temperature whereby the mixture is pourable onto a wound to be treated when below said transition temperature, and in contact with the skin setting to form a non-flowable gelatinous wound dressing.

The wound dressing compositions in accordance with this invention are semi-viscous pourable fluids at relatively low temperatures. They may therefore be applied, from a suitable container, which preferably will have been kept refrigerated (about 3°-8°C) at least immediately prior to use, by being poured onto the open wound, to take up the contours of the wound. To ensure satisfactory pourability at the time of use it is preferred that the viscosity of the aqueous mixture should be from 50 to 250 poise (Brookfield LV viscometer with No. 4 spindle) immediately below its sol-gel transition temperature. Further, it is preferred that the sol-gel transition temperature should lie between 10°-25°C, more preferably 15°-20°C. With a transition temperature lower than 10°C, there is a risk that the composition will set too quickly after removal from the refrigerator thus making it difficult, if not impossible, to apply to a wound. Conversely, with a transition temperature above 25°C, the composition can take an undesirable time to set post-application, or in some circumstances may not set at all.

After being applied to the wound site, the composition forms what we term a "gelatinous-film" or "gel-film" as its temperature is raised to ~20°-25°C in contact with the patient's tissue, and this gel-film, unlike currently available gel dressings, does not normally require an additional dressing to hold it in place, although, of course, such an additional dressing may be applied if considered expedient by the physician. The characteristics of this gel-film vary somewhat, depending on the quantities of the gel-forming and film-forming ingredients, and on the possible presence of other ingredients. Indeed, it is a particular advantage of this invention that by varying the amounts of the ingredients a range of different wound dressings can be provided to more clearly suit the requirements of different types of wounds.

More particularly, the amounts of the gel-forming and film-forming ingredients can be varied so as to emphasise the film-forming properties or alternatively the gel-forming properties of the compositions.

Thus, it is possible, for instance, to formulate the wound composition so that, on setting, it forms a tough, flexible, coherent film on the skin surface which provides a barrier to microorganisms and good resistance to mechanical stresses. Such a formulation will be particularly suitable for treating superficial wounds such as dermabrasions, skin-graft donor sites, shallow burns, etc. More particularly, compositions which set to form such flexible films can be formed from mixtures comprising from 1% to 5%, preferably 2% to 3%, by weight of the composition of the gel-forming component (a), and from 5% to 20%, preferably 8% to 12%, by weight of the composition of the film-forming component (b).

In such compositions, the gel-forming component (a), although present in relatively low concentrations, nonetheless serves to increase significantly the rate of formation of the gelatinous-film so that it sets in a reasonably short time, without impairing the strength or toughness of the set film.

By means of the present invention, and in preferred instances, it is possible to provide wound dressing compositions which set on tissue to form films which:

(1) are strong and durable, yet conformable and elastic, to isolate the wound and provide a barrier to microbial invasion and mechanical stresses;

(2) allow gaseous exchange and exit of water vapour, but retain sufficient water to maintain a high humidity at the wound dressing interface;

(3) can be readily washed away so that they can be removed without trauma to the patient; and

(4) are usually colourless and translucent, allowing visual examination of the wound through the dressing itself.

In addition, of course, these compositions, indeed like all the compositions of this invention, must be non-antigenic and non-toxic, be sterilizable and be stable on storage.

An alternative approach, emphasising the gel-former rather than the film-former, provides a composition which sets to a rigid gel, and is particularly suitable for the treatment of deep-seated wounds such as leg ulcers and deep burns. Such a gel is non-flowable; for instance, the meniscus of the product will not move when it is tilted through 90°. It typically has the consistency of a firm "jelly" such as that produced when gelatin in water is allowed to set, but mechanically stronger. More particularly, compositions which set to form such a rigid gelatinous mass can be formed from mixtures comprising from 5% to 30% by weight, preferably 7% to 20%, by weight of the composition of the gel-forming component (a), and from 1% to 15%, preferably 7% to 12%, by weight of the composition of the film-forming component (b).

In these compositions, the film-forming component (b) enhances the rate of gelation of the composition, controls its sol-gel transition and, in most instances can actually increase the gel strength at skin temperatures.

By means of the present invention, and in preferred instances, it is possible to provide wound dressing compositions which set in contact with a deep-seated wound to form rigid gels which:

(1) readily conform to the wound contours and fill the wound cavity;

(2) are gas and water-vapour permeable whilst providing a barrier to microorganisms and mechanical stresses;

(3) have the ability to absorb excess exudate and toxic substances at the wound site;

(4) have the ability to provide thermal insulation of the wound;

(5) are easily washed away so that they can be removed without trauma to the patient;

(6) are usually colourless and transparent, allowing visual observation of the wound through the dressing itself; and

(7) allow aspiration of exudate through the dressing, e.g. by means of a syringe, the access site through the dressing then resealing itself.

In addition, there are the same requirements that the formulations should be non-toxic and non-antigenic, stable on storage and sterilizable.

Of course, it will be understood that the wound dressing compositions of this invention may be formulated so as to have properties intermediate between those of the two types of formulation just referred to.

The concentrations of the components of the composition, as well as the presence of optional ingredients will also affect its gel-setting time to some extent, but this property is also governed by other factors as well, for instance the volume-to-surface area relationship which will affect the heat transfer into the gel and hence the gel setting time, and also the temperature at which the composition is dispensed. Desirably, the gel setting time will be from about 1 to 15 minutes.

The gel-forming and film-forming ingredients of the wound dressing compositions of the present invention may be provided by the same compounds as described in relation to the drug delivery compositions. Again, we often prefer to employ Pluronic F127 as the gel former and Natrosol 250L as the film-forming component.

The aqueous vehicle will normally consist of water alone, although in some instances it may also comprise minor amounts e.g. up to 20% w/w, of a miscible, non-toxic and non-antigenic organic liquid e.g. polyethylene glycol, for example in order to improve the conformability and plasticity of compositions containing relatively larger concentrations of the film-forming component.

As indicated above, other ingredients may optionally be included in the wound treatment compositions of this invention in order to modify their properties. For instance, we have found that incorporating a plasticizer enhances the elasticity of the set film and the adherency of the film to the wound surface. Suitable plasticizers include polyethylene glycol, glycerol, sorbitol, ethanolamine and calcium chloride. We currently, prefer to incorporate polyethylene glycol, more especially PEG 400 in amounts of from 1% to 10% by weight of the composition in those compositions which are formulated so as to be adapted for the treatment of superficial wounds. However, we prefer to omit a plasticizer from compositions formulated for the treatment of deep-seated wounds.

In compositions which have a relatively high proportion of the gel-former, it is often preferred to incorporate as a stabilizing agent an hydroxy- or polyhydroxy-compound in order to enhance the gel strength, lower the sol-gel transition temperature and reduce the risk of phase separation occurring during storage. Suitable stabilizing agents include glycerol, propylene glycol, ethanolamine, mannitol and sorbitol. We currently prefer to incorporate sorbitol in an amount of from 1% to 10% by weight of the composition. It is, of course, necessary that the stabilizing agent should be non-toxic and non-antigenic.

Another optional ingredient is a preservative, to protect the composition from microbial contamination during manufacture and storage and during the time it is on a patient. However, the presence of a preservative may, in some circumstances, adversely affect the wound-healing process and generally therefore we prefer to omit it. If a preservative is to be incorporated, benzyl alcohol in a concentration of about 0.5 to 2% by weight based on the weight of the composition, preferably about 1%, has been found to be satisfactory, but other suitable preservatives include methyl and propyl parabens, chlorbutanol, benzalkonium chloride, ascorbate, sodium thiosulfate, sodium bisulfite, thiomersal and 2-phenoxyethanol. Benzyl alcohol also has the effect of acting as a stabilizing agent, being a hydroxy-containing compound, and therefore lowers the gelation temperature. This property therefore needs to be taken into account in determining the amounts of the other ingredients when benzyl alcohol is included.

In general, the pH of the present compositions can range from pH 3 to pH 9. Gelling temperature and strength may be affected to some extent by the pH of the composition, although we have found that a pH within the range of 4-7.5 generally has no significant effect on either gel strength or gel temperature, and we therefore prefer to operate within this range, since also it provides an inhibitory effect on bacterial cell growth. Most desirably, the pH of the present compositions is from 4.5 to 6.5, such a pH range providing an inhibitory effect on bacterial cell growth. If necessary, the pH of the compositions can be adjusted to achieve a desired level using any pharmaceutically acceptable acid or base. Sodium hydroxide or ethanolamine, for instance, may be used to raise the pH, whilst hydrochloric acid is suitable for reducing pH.

A particular embodiment of the present invention is a composition which can serve both as a wound-dressing and which also incorporates a pharmaceutically active ingredient for delivery to the wound site, e.g. antibacterial agents for the control of infection, chemotactic agents to induce the migration of normal fibroblasts and/or agents to promote rapid re-epithelialisation.

It is of course necessary that the wound dressing compositions of this invention should be sterile at the time of administration. We prefer to sterilize those compositions containing relatively smaller amounts of the gel-forming component by gamma irradiation, suitably after filling the compositions into flexible sachets for dispensing. On the other hand, those compositions containing relatively larger amounts of the gel-former are not successfully sterilized by this technique; for the latter compositions, we prefer to sterilize the hydroxyethylcellulose, or other film-forming ingredient, using gamma irradiation, and to sterile filter the solution containing the gel-former and any optional ingredients, whereafter the sterile film-former is added to the sterile solution under aseptic conditions. Heating in an autoclave using a standard pharmacopoeial cycle is another method which can sometimes be used to sterilize the compositions but we have found that this technique may cause an unacceptable decrease in gel strength and viscosity.

The wound dressing compositions of this invention preferably are filled into flexible sachets for dispensing. However, containers other than sachets, eg aerosol spray containers or tube dispensers can, of course, also be used. The filled sachets or other containers can, if desired, be stored under refrigeration, but it appears that storage at ambient temperatures does not adversely affect the reversible gelation properties of the compositions, although if the product has been stored at a temperature above its sol-gel transition temperature it will then need to be cooled to below this temperature prior to administration.

The preferred wound treatment compositions of this invention have a number of advantages. In particular, the compositions possess a combination of both film- and gel-forming properties, which can be varied by appropriate selection of the components, and amounts thereof, as described above, so as to render them particularly suitable for different indications, e.g. treatment of superficial or deep-seated wounds. Moreover, they offer the practical advantage of forming a gelatinous-film in situ at the wound site, being easy both to apply and remove with the minimum of trauma to the patient. In the treatment of burns, the cooling effect which occurs in situ as the composition gels can be soothing to the patient. The gelatinous-film which forms on setting itself has a number of important advantages as a wound dressing, as hereinbefore indicated.

The invention is illustrated by the Examples which follow and in which all percentages are given in (w/w)% unless otherwise indicated.

All viscosities were measured using a Brookfield viscometer, model LV; spindle No. 4, at the temperature indicated.

Examples 1-14 illustrate drug delivery compositions of this invention and Examples 15-20 illustrate wound dressing compositions of this invention.

## Example 1

This example illustrates the preparation of ophthalmic compositions containing the non-steroidal anti-inflammatory drug biphenyl acetic acid (BPAA) for delivery to the eye.

The following four test compositions were prepared, by following the general procedure described hereinabove.

| | 1 | 2 | 3 | 4 |
|---|---|---|---|---|
| BPAA (ethanolamine salt) | 0.5% | 0.5% | 0.5% | 0.5% |
| Pluronic F127 | 8.0% | 7.0% | 7.0% | 8.0% |
| Hyroxyethylcellulose[1*] | 8.0% | 7.0% | 7.0% | 8.0% |
| Sorbitol | 2.5% | 2.5% | 5.0% | 5.0% |
| Benzalkonium Chloride | 0.01% | 0.01% | 0.01% | 0.01% |
| Borate buffer[2*] | 2.0% | 2.0% | 2.0% | 2.0% |
| Water to | 100.0% | 100.0% | 100.0% | 100.0% |
| pH (adjusted with 10% ethanolamine solution) | 8.3 | 8.3 | 8.3 | 8.3 |
| Sol-gel Transition Temperature | 26°C | 34°C | 32°C | 25°C |
| Solution Viscosity | 1040 cps, 19°C | 815 cps, 19°C | 615 cps, 19°C | 1165 cps, 19°C |
| Gel Viscosity* | 15060 cps, 30°C | 6200 cps, 36°C | 6325 cps, 35°C | 17760 cps/30°C |
| t50% (minutes)[3*] | 136 | 50 | 47 | 75 |
| t90% (minutes)[4*] | 304 | 156 | 134 | 197 |

[1*] Natrosol 250 L Pharm
[2*] This is 2% w/w of the following buffer solution; 50 ml of a 0.025 M solution of $Na_2B_4O_7.10H_2O$ (9.525 g/1), 17.7 ml of a 0.1 M solution of HCl and 32.3 ml of $H_2O$.
[3*] time taken for 50% of drug to be released
[4*] time taken for 90% of drug to be released

All four test ophthalmic compositions can be administered as liquids, for example by means of an eye dropper, if they are cooled to below their sol-gel transition temperatures.

The example demonstrates that the gel viscosity increases, whilst the sol-gel transition temperature decreases, with an increase in the concentrations of the Pluronic F127 and hydroxyethylcellulose, and with an increase in the concentration of the sorbitol.

The t50% and t90% values for the rate of drug release given in the table demonstrate that this rate can be controlled by varying the formulation of the composition. Thus, increasing the polymer (Pluronic 127/hydroxyethylcellulose) content but maintaining the same sorbitol content results in a retardation of the rate of BPAA released from the gel, probably as a result of the gel having an increased viscosity. However, for the same polymer concentrations, with an increase in sorbitol concentration there is an increase in the rate of BPAA released notwithstanding the increase in gel viscosity.

The dissolution/release tests were conducted on the four test compositions using the dissolution apparatus of design and specification described in the 1988 British Pharmacopoeia, Appendix XIID under 'Apparatus I'. Each vessel contained 900 ml of isotonic phosphate buffer (pH 7.4) dissolution medium prepared from $Na_2HPO_4$ 7.57 g/litre, $NaH_2PO_4.2H_2O$ 2.08 g/litre and NaCl 4.4 g/litre. Agitation was provided by a drive shaft with basket attachment positioned in the dissolution medium 2.5 cm above the bottom of the vessel and rotated at 50 rpm.

Approximately 5 g of the test formulation was warmed to 30°C to gel the product, and then accurately weighed into the dissolution basket with its base lined with Visking tubing. The basket containing gel, was carefully attached to the drive shaft and lowered into the dissolution medium.

Samples (15 ml) were withdrawn periodically at known time intervals and filtered through a 0.45 μm Acrodisc™ membrane filter prior to the determination of BPAA concentration spectrophometrically at 254 nm. Beer-Lambert plots for BPAA in the presence and absence of gel components demonstrated that there was negligible interference from the gel components, and that the UV response was linear over a concentration range from 0.006 to 0.03 mg/ml (r = 0.9996).

Example 2

This example illustrates the preparation of another ophthalmic composition, in this case for delivery of chloramphenicol, a broad spectrum antibiotic, to the eye.

The following composition was prepared, again by following the general procedure described hereinabove.

| Chloramphenicol BP | 0.5% |
|---|---|
| Pluronic F127 | 6.0% |
| Hydroxyethylcellulose | 6.0% |
| Sorbitol | 3.0% |
| Benzalkonium Chloride | 0.01% |
| Sodium Acetate | 0.3% |
| Water to | 100.0% |

A clear, colourless solution, with the following properties was formed.

| pH | 4.94 |
|---|---|
| Sol-gel Transition Temperature | 21-23°C |
| Solution Viscosity | 760 cps, 13°C |
| Gel Viscosity | 5470 cps, 30°C |

Similar ophthalmic compositions but containing as the pharmacologically active component in place of chloramphenicol (a) gentamicin sulphate 0.3%, (b) lignocaine HCl 4.0%, (c) nystatin 1.0% and (e) acetazolamide 3.5%, were also prepared. Nystatin and acetazolamide, being water-insoluble, were incorporated as suspensions by dispersing the drug in the vehicle using a mixer.

Example 3

This example illustrates the preparation of a composition containing metronidazole, an antibacterial agent for topical delivery to the skin.

Following the general procedure hereinabove described, the following composition was prepared:

| Metronidazole BP | 0.5% |
|---|---|
| Pluronic F127 | 10.0% |
| Hydroxyethylcellulose | 10.0% |
| Sorbitol | 6.0% |
| Water to | 100.0% |

A clear solution with a pale yellow tinge was obtained, with the following properties:

| pH | 5.24 |
|---|---|
| Sol-gel Transition Temperature | 15°-17°C |
| Solution Viscosity | 15,150 cps/8°C |
| Gel Viscosity | 897,000 cps/25°C |

As will be noted, in this case a more rigid gel was obtained above the sol-gel transition temperature than in the previous Examples, as indicated by the values of the gel viscosity of the composition. This was achieved by the use of higher concentrations of the gel-forming component Pluronic F127 and of the film-forming component hydroxyethylcellulose. It is an advantage that the gel be more rigid for topical application to the skin because it will need to be mechanically stronger to withstand the physical stresses to which it may be exposed and which will tend to displace it from the site of application. Moreover, the composition had a somewhat lower sol-gel transition temperature than the compositions of Examples 1 and 2. These characteristics are desirable for topical use, to ensure that the composition sets in contact with the patient's tissues.

Similar compositions but containing as the pharmacologically active component in place of metronidazole (a) gentamicin 0.3%, (b) lignocaine hydrochloride 2.0% and (c) BPAA 3.0%, were also prepared.


## Example 4


This example illustrates the preparation of a composition containing streptokinase-streptodornase ("Varidase"), a debriding agent, for topical application for the treatment of suppurating surface lesions such as ulcers, pressure sores, burns and the like.

Following the general procedure described hereinabove, the following composition was prepared:

| "Varidase"* | 0.2%** |
|---|---|
| Pluronic F127 | 10.0% |
| Hydroxyethylcellulose | 10.0% |
| Sorbitol | 6.0% |
| Water to | 100.0% |

* "Varidase" is a proprietary name for streptokinase-streptodornase
**Equivalent to 6200 units of streptokinase and 4800 units of streptodornase per ml

An opaque solution/suspension, with the following properties, was obtained:

| pH | 6.6 |
|---|---|
| Sol-gel Transition Temperature | 15°-17°C |
| Viscosity of Solution | 14,300 cps/8°C |
| Viscosity of Gel | 382,000 cps/25°C |

Since "Varidase" is unstable in aqueous compositions, it is an example of a pharmacologically active compound which should not be incorporated into the delivery composition until just before use. For this purpose, it can be supplied as a freeze-dried powder for reconstitution with the delivery composition immediately before administration.

The composition of this example may be used as a wound debriding formulation.

## Example 5

This example illustrates the preparation of a composition containing gentamicin for intramuscular or subcutaneous delivery of the drug in the treatment of septicaemia or severe systemic infections.

Following the general procedure described hereinabove, the following composition was prepared:

| Gentamicin Sulphate | 4.0% |
|---|---|
| Pluronic F127 | 6.0% |
| Hydroxyethylcellulose | 6.0% |
| Sorbitol | 3.0% |
| Water | 100.0% |

A clear colourless solution, with the following properties, was obtained:

| pH | 5.20 |
|---|---|
| Sol-gel Transition Temperature | 27-30°C |
| Solution Viscosity | 1,100 cps/21°C |
| Gel Viscosity | 43,900 cps/31°C |

Similar intramuscular/subcutaneous compositions but containing as the pharmacologically active component in place of gentamicin (a) BPAA 1.0%, (b) lignocaine hydrochloride 2.0% and (c) piperacillin 20%, were also prepared. Since piperacillin is relatively unstable in aqueous media, it should be incorporated into the delivery composition just prior to injection.

## Example 6

This example illustrates the preparation of a composition containing lignocaine, a local anaesthetic, for buccal application in the treatment of mouth ulcers and oral abrasions.

Following the general procedure described hereinabove, the following composition was prepared:

| Lignocaine HCl | 0.6% |
|---|---|
| Pluronic F127 | 10.0% |
| Hydroxyethylcellulose | 10.0% |
| Sorbitol | 6.0% |
| Cetyl Pyrridinium Chloride | 0.02% |
| Water to | 100.0% |

A clear colourless solution, with the following properties, was obtained:

| pH | 5.12 |
|---|---|
| Sol-gel Transition Temperature | 16°-17°C |
| Solution Viscosity | 18,050 cps/8°C |
| Gel Viscosity | 1,446,000 cps/25°C |

The gel exhibits muco-adhesive properties, thus adhering to the site of the mouth ulcer or abrasion, whereby the action of the lignocaine is prolonged, to give longer pain relief to the patient.

Other pharmacologically active substances such as BPAA, methyl salicylate and antibiotics such as minocycline, can be similarly formulated to provide compositions for relieving pain and/or preventing infection in the mouth or buccal area.

## Example 7

This example illustrates the preparation of a composition containing hydrocortisone for the treatment of gastro-intestinal disorders by oral delivery.

Following the general procedure described hereinabove, the following composition was prepared:

| | |
|---|---|
| Hydrocortisone sodium succinate | 1.34% |
| Sorbitol | 6.00% |
| Pluronic F127 | 10.00% |
| Hydroxyethylcellulose | 10.00% |
| Water to | 100.00% |

A translucent, colourless solution/suspension, containing the equivalent of 1.00% hydrocortisone, with the following properties was obtained:

| | |
|---|---|
| pH | 6.95 |
| Sol-gel transition temperature | 15-17 $^\circ$C |
| Solution viscosity | 5130 cps/8 $^\circ$C |
| Gel viscosity | 729000 cps/25 $^\circ$C |

## Example 8

This example illustrates the preparation of a composition containing lignocaine, for vaginal/rectal delivery to achieve surface anaesthesia of the tissue.

Following the general procedure described hereinabove, the following composition was prepared:

| | |
|---|---|
| Lignocaine HCl | 1.0% |
| Pluronic F127 | 10.0% |
| Hydroxyethylcellulose | 10.0% |
| Sorbitol | 6.0% |
| Chlorhexidine Acetate | 0.05% |
| Water to | 100.0% |

A clear colourless solution, with the following properties, was obtained:

| | |
|---|---|
| pH | 5.5 |
| Sol-gel Transition Temperature | 20 $^\circ$-21 $^\circ$C |
| Solution Viscosity | 19,950 cps/8 $^\circ$C |
| Gel Viscosity | 1,266,000 cps/25 $^\circ$C |

Similar compositions but containing as the pharmacologically active component in place of lignoconidazole 10.0% and (b) nystatin 1.0% (both as suspensions) were also prepared.

A spermicidal composition was also similarly prepared, containing nonoxynol 9 as the active in a concentration of 5%.

## Example 9

This illustrates the preparation of a composition for topical delivery of biphenylacetic acid (BPAA) using polyvinyl alcohol as the film-forming component.

Following the general procedure described hereinabove, the following composition was prepared.

| BPAA | 3.0% |
|---|---|
| Pluronic F127 | 20.0% |
| Polyvinyl alcohol (MW = 26,000) | 5.0 |
| Benzyl alcohol | 1.0 |
| Water to | 100.0% |

A clear gel, with the following properties, was obtained:

| pH | 6.7 |
|---|---|
| Sol-gel transition temperature | $18°$-$19°$ C |
| Solution viscosity | 12,010cps/$8°$ C |
| Gel viscosity | 351,060cps/$25°$ C |

## Example 10

This illustrates the preparation of a composition for topical delivery of metronidazole to the skin.

Following the general procedure described hereinabove, the following composition was prepared:

| Metronidazole | 0.5% |
|---|---|
| Pluronic F127 | 18.0% |
| Polyvinyl alcohol (MW = 26,000) | 7.5% |
| Water to | 100.0% |

A clear gel, with a pale yellow tinge, was formed. The solution had the following properties:

| pH 7.5 (adjusted with NaOH) | |
|---|---|
| Sol-gel transition temperature | $19°$-$20°$ |
| Solution viscosity | 15,500cps/$8°$ C |
| Gel viscosity | 274,000cps/$25°$ C |

## Example 11

This example illustrates the preparation of a composition for the delivery of gentamicin sulphate by injection.

Following the general procedure described hereinabove, the following composition was prepared:

| Gentamicin sulphate | 4.00% |
|---|---|
| Hydroxyethylcellulose | 2.00% |
| Pluronic F108 | 15.00% |
| Water to | 100.00% |

A translucent, colourless solution with the following properties was obtained:

| pH | 5.33 |
|---|---|
| Sol-gel transition temperature | 40-45° C |
| Solution viscosity | 800 cps/9° C |
| Solution viscosity | 6,200 cps/37° C |
| Gel viscosity | 53,300 cps/42° C |

In this case, the composition was a low viscosity liquid which did not form a gel at physiological temperatures. It is thus suitable for injection by the intravenous, intramuscular, subcutaneous or other routes for the treatment of septicaemia or other severe systemic infections.

## Example 12

This example illustrates the preparation of a composition for the vaginal or rectal delivery of metronidazole, to prevent or treat local or systemic microbial infections.

Following the general procedure described hereinabove, the following composition was prepared:

| Metronidazole | 0.50% |
|---|---|
| Polyethylene glycol 14000 | 0.50% |
| Hydroxyethylcellulose | 2.50% |
| Pluronic F127 | 15.00% |
| Water to | 100.00% |

A clear, colourless solution with the following properties was obtained:

| pH | 6.35 |
|---|---|
| Sol-gel transition temperature | 34-39° C |
| Solution viscosity | 130 cps/20° C |
| Gel viscosity | 5950 cps/37° C |
| Gel viscosity | 45600 cps/42° C |

This composition, suitable for use as a suppository, has a broad sol-gel transition temperature owing to the gentle rise in viscosity between 34° C and 39° C. Such a composition should be heated to between 40° C and 42° C to form a gel rigid enough for insertion, but once in the body, it reverts to a more fluid state to aid absorption (in the preferred manner of existing suppository types).

## Example 13

This example illustrates the preparation of a composition containing a dye for ophthalmic use, to aid the diagnosis of damage to the eye surface.

Following the general procedure described hereinabove, the following composition was prepared:

| | |
|---|---|
| Rose Bengal | 1.00% |
| Methyl paraben | 0.20% |
| Propyl paraben | 0.05% |
| Hydroxyethylcellulose | 7.00% |
| Pluronic F127 | 7.00% |
| Sorbitol | 5.00% |
| Water to | 100.00% |

A solution, coloured deep pink, was obtained which had the following properties:

| | |
|---|---|
| pH | 6.11 |
| Sol-gel transition temperature | 25-27 °C |
| Solution viscosity | 820 cps/15 °C |
| Gel viscosity | 13600 cps/31 °C |

## Example 14

This example illustrates the preparation of a composition containing a radio-opaque contrast medium. Following the general procedure described hereinabove, the following composition was prepared:

| | |
|---|---|
| Sodium diatrizoate | 4.00% |
| Hydroxyethylcellulose | 7.00% |
| Pluronic F127 | 7.00% |
| Sorbitol | 5.00% |
| Water to | 100.00% |

A translucent, colourless solution was obtained with the following properties:

| | |
|---|---|
| pH | 6.01 |
| Sol-gel transition temperature | 24-26 °C |
| Solution viscosity | 2900 cps/8 °C |
| Gel viscosity | 38350 cps/30 °C |

This composition is suitable for gastrointestinal use (either by oral or rectal administration), or, by retrograde instillation, in the urinary system.

## Example 15

A wound dressing of the following composition was prepared.

| Pluronic F127 | 2.5% | 10g |
|---|---|---|
| Hydroxyethylcellulose* | 10.0% | 40g |
| Benzyl alcohol | 1.0% | 4g |
| PEG 400 | 4.0% | 16g |
| Purified water to | 100.0% | 330g |

* "Natrosol 250L"

Hydroxyethylcellulose (40g) was slowly and carefully added to purified water (330g) at room temperature with continuous gentle agitation to produce a translucent, colourless solution. PEG 400 (16g) and benzyl alcohol (4g) were added and thoroughly mixed with the hydroxyethylcellulose solution, and this was transferred to an ice-bath and cooled to 3-4° C. Pluronic F127 (10g) was slowly added to the cold solution with gentle mixing, and allowed to hydrate and dispersed overnight at 3-4° C to produce a clear colourless solution, the film former product.

The final solution had a pH of 4.9, which was adjusted to pH 6.4 using 1N NaOH.

Aliquots of the resulting solution were filled in to 3 layer laminate pouches (polyester foil/polyethylene), heat sealed and terminally sterilized by gamma-irradiation (2.5 MRads).

| Viscosity*** at 3-4° C | 86.1 poise |
|---|---|
| Viscosity*** at 25° C | 134.4 poise |
| Sol-Gel transition temperature | 22°-25° C |

*** measured using a Brookfield Model LV viscometer with a No. 4 spindle

This product does not gel strongly, but forms a tough, flexible, transparent film which dries on the skin in approximately 15 minutes

## Example 16

A further wound dressing was prepared, having the following composition:

| Pluronic F127 | 10% | 40g |
|---|---|---|
| Hydroxyethylcellulose* | 10% | 40g |
| Benzyl alcohol | 1% | 4g |
| Purified water to | 100% | 316g |

* "Natrosol 250L"

The composition was prepared by the method described in Example 15 up to (but not including) the step of filling the resulting solution into pouches and sterilizing the product.

22

| Final pH of formulation; unadjusted | 5.24 |
| Viscosity** at 3°-4°C | 165 poise |
| Viscosity** at 25°C | 9540 poise |
| Sol-Gel Transition Temperature | 16°-18°C |
| Gel-setting time | 4-5 minutes |

** measured using Brookfield LV viscometer with a No. 4 spindle

## Example 17

A further wound dressing was prepared, having the following composition:

| Pluronic F127 | 11% | 44g |
| Hydroxyethylcellulose* | 10% | 40g |
| Purified water to | 100% | 316g |

*"Natrosol 250L"

The composition was prepared by the method described in Example 16.

| Final pH of formulation; unadjusted | 6.17 |
| Viscosity** at 3°-4°C | 141.8 poise |
| Viscosity** at 25°C | 12,360 poise |
| Sol-Gel Transition temperature | 15°-18°C |
| Gel setting time | 4-5 minutes |

** measured using Brookfield LV viscometer with a No. 4 spindle

It was found that with this composition phase separation tended to occur on long term storage.

## Example 18

A further wound dressing composition was prepared by the method described in Example 16 from the following ingredients:

| Pluronic F127 | 10% | 40g |
| Hydroxyethylcellulose* | 10% | 40g |
| Sorbitol | 6% | 24g |
| Purified Water to | 100% | 296g |

*"Natrosol 250L"

The properties of the resulting wound dressing were as follows:

23

EP 0 386 960 A2

| Final pH of formulation, unadjusted | 5.31 |
|---|---|
| Viscosity*** at 3°-4° C | 185 poise |
| Viscosity*** at 25° C | 9010 poise |
| Sol-gel transition temperature | 16-17° C |
| Gel-setting time | 4-5 minutes |

*** measured using Brookfield LV viscometer with a No. 4 spindle

The wound dressing composition of this example did not suffer from the phase separation encountered on long term storage with the composition of Example 17, whilst retaining similar properties to those of the product of Example 17.

## Example 19

A sterile wound dressing composition, of the same formulation as in Example 18, was prepared as follows:

Sorbitol (24g) was dissolved in purified water (296g) at room temperature with continuous agitation to produce a clear colourless solution. Pluronic F127 (40g) was slowly added to the sorbitol solution with gentle mixing and hydration at room temperature. This step took about 1 to 2 hours to ensure complete dispersion of the Pluronic F127. The resulting solution was sterilized by filtration through a 0.22 micron filter, and hydroxyethyl cellulose (40g), which had previously been sterilized by gamma-irradiation (2.5 MRads), was added to the solution under aseptic conditions to complete the formulation. Aliquots of the resulting solution were filled into sterile sachets, at a temperature of 3 to 8° C to prevent gelling.

The properties of the resulting, sterile, wound dressing were as follows:

| Final pH of formulation, unadjusted | 5.36 |
|---|---|
| Viscosity*** at 3°-4° C | 180.5 poise |
| Viscosity*** at 25° C | 6500 poise |
| Sol-gel transition temperature | 15°-16° C |
| Gel-setting time | 4-5 minutes |

*** measured using Brookfield LV viscometer with a No. 4 spindle

Comparing the properties of the two products of Examples 18 and 19, it will be noted that the sterilization of the hydroxyethylcellulose has resulted in the viscosity at 25° C falling from 9010 poise to 6500 poise, but that the other properties remained substantially the same. The product of this example was, however, still a firm gel at 25° C and therefore could be used eg on deep-seated wounds.

## Example 20

This example illustrates a wound dressing in accordance with this invention which includes an antibacterial agent.

Following the method of Example 16, a wound dressing of the following composition was prepared.

24

EP 0 386 960 A2

| Pluronic F127 | 10% | 40g |
|---|---|---|
| Hydroxyethylcellulose* | 10% | 40g |
| Chlorhexidine acetate | 0.5% | 2g |
| Purified water to | 100% | 318g |

* Natrosol 250L

The properties of this wound dressing were as follows:

| Final pH of formulation; unadjusted | 6.1 |
|---|---|
| Viscosity** at 3°-4° C | 183 poise |
| Viscosity** at 25° C | 9300 poise |
| Sol-gel transition temperature | 18°-20° C |

** measured using Brookfield LV viscometer with a No. 4 spindle

The above Examples were all conducted on a relatively small scale. We have found that on scaling-up, the final viscosities of the compositions tend to become lower than those values reported for the small scale experiments, probably because of the increased mixing efficiency which is attained on the larger scale. However, the other physical properties remain essentially constant.

## Claims

1. A drug delivery composition, comprising an aqueous vehicle containing (a) from 1% to 30% by weight of one or more compounds having reversible thermosetting gel properties, and (b) from 1% to 15% by weight of one or more compounds, compatible with component (a), having film-forming properties, and the composition having a sol-gel transition temperature within the range 0° to 50° C.

2. A composition according to Claim 1 and comprising also from 0.001% to 35% by weight of at least one compound having pharmacological or diagnostic activity.

3. A composition according to Claim 1 or Claim 2 and having a sol-gel transition temperature within the range 10° to 40° C.

4. A composition according to Claim 3 and having a sol-gel transition temperature within the range 15°-35° C.

5. A composition according to Claim 3, for application topically to the body tissues of a patient, and having a sol-gel transition temperature of 15°-20° C.

6. A composition according to Claim 3, for application ophthalmically, and having a sol-gel transition temperature of 20°-26° C.

7. A composition according to any preceding claim, and having a viscosity of 0.5 to 250 poise (Brookfield Model LV, No. 4 spindle) immediately below its sol-gel transition temperature.

8. A composition according to any preceding claim, comprising (a) from 3% to 30% by weight of said one or more compounds having reversible thermosetting gel-properties, and (b) from 3% to 15% by weight of said one or more compounds having film-forming properties, said composition on setting forming a gel.

9. A composition according to Claim 8, comprising from 5% to 15% by weight of said component (a) and from 5% to 10% by weight of said component (b).

10. A composition according to any one of Claims 1-7, comprising (a) from 1% to 10% by weight of said one or more compounds having reversible thermosetting gel-properties, and (b) from 1% to 10% by weight of said one or more compounds having film-forming properties, said composition on setting forming a gel.

11. A composition according to Claim 10, comprising from 4% to 7% by weight of said component (a) and from 4% to 7% by weight of said component (b).

12. A composition according to any one of Claims 1-7, comprising (a) from 1% to 5% by weight of said one or more compounds having reversible thermosetting gel properties, and (b) from 5% to 15% by weight of said one or more compounds having film-forming properties, said composition on setting forming a gel.

13. A composition according to Claim 12, comprising from 2% to 3% by weight of said component (a)

25

and from 8% to 12% of said component (b).

14. A composition according to any preceding claim, wherein said component (a) comprises one or more compounds selected from block copolymers of polyoxyethylene-polyoxypropylene and tetra-substituted derivatives of ethylene diamine where the substituents are block copolymers of polyoxyethylene-polyoxypropylene.

15. A composition according to Claim 14, wherein said component (a) comprises a polyoxyethylene-polyoxypropylene block copolymer having an average molecular weight of about 11,500.

16. A composition according to any preceding claim, wherein said component (b) comprises one or more compounds selected from hydroxyethylcellulose, hydroxypropylmethylcellulose and polyvinyl alcohol.

17. A composition according to Claim 16, wherein said component (b) is a low viscosity grade hydroxyethylcellulose.

18. A composition according to any preceding claim, wherein said aqueous vehicle is water.

19. A composition according to any preceding claim, comprising also a hydroxy- or polyhydroxy-compound to improve the physical stability of the composition.

20. A composition according to Claim 19, wherein said stability-improving compound is selected from benzyl alcohol, glycerol, propylene glycol, ethanolamine, mannitol and sorbitol.

21. A composition according to Claim 20, wherein said composition comprises from 0.5% to 20% by weight of sorbitol.

22. A composition according to any preceding claim which has been sterilized.

23. A wound dressing composition comprising an aqueous vehicle containing (a) from 1% to 30% by weight of the composition of one or more compounds having reversible thermosetting gel properties, and (b) from 1% to 20% by weight of the composition of one or more compounds, compatible with component (a) having film-forming properties, and the composition having a sol-gel transition temperature within the range $10°$ to $30°$ C, a viscosity of not greater than 250 poise, as determined by a Brookfield viscometer (Model LV with No. 4 spindle), at a temperature immediately below its sol-gel transition temperature whereby the mixture is pourable onto a wound to be treated when below said transition temperature, and in contact with the skin setting to form a non-flowable gelatinous wound dressing.

24. A wound dressing composition according to Claim 23, having a sol-gel transition temperature of from $10°$ to $25°$ C.

25. A wound dressing composition according to Claim 24, having a sol-gel transition temperature of from $15°$ to $20°$ C.

26. A wound dressing composition according to any one of Claims 23-25, having a viscosity of 50 to 250 poise, as determined by a Brookfield viscometer (Model LV with No. 4 spindle) immediately below its sol-gel transition temperature.

27. A wound dressing composition according to any one of Claims 23-26, wherein the gel-forming component exhibits reversible thermosetting gel properties at a temperature within the range $10°$-$45°$ C.

28. A wound dressing composition according to any one of Claims 23-27, comprising 1% to 5% by weight of the composition of the gel-forming component (a) and from 5% to 20% by weight of the composition of the film-forming component (b), the composition setting in contact with the skin to form a coherent film thereon.

29. A wound dressing composition according to Claim 27 and including also a plasticizer.

30. A wound dressing composition according to Claim 29, comprising from 2% to 3% by weight of the composition of the gel-forming component (a), from 8% to 12% by weight of the composition of the film-forming component (b) and from 1% to 10% by weight of the composition of the plasticizer.

31. A wound dressing composition according to Claim 29 or Claim 30, wherein the plasticizer is polyethylene glycol.

32. A wound dressing composition according to any one of Claims 23-27, comprising from 5% to 30% by weight of the composition of the gel-forming component (a) and from 1% to 15% by weight of the composition of the film-forming component (b), the composition setting in contact with the skin to form a rigid gel.

33. A wound dressing composition according to Claim 32, comprising from 7% to 20% by weight of the composition of the gel-forming component (a) and from 7% to 12% by weight of the composition of the film-forming component (b).

34. A wound dressing composition according to Claim 32 or Claim 33, comprising also an hydroxy- or polyhydroxy-compound as a stabilizing agent.

35. A wound dressing composition according to Claim 34, wherein the stabilizing agent is sorbitol.

36. A wound dressing composition according to Claim 34 or Claim 35, wherein the stabilizing agent is present in an amount of from 1% to 10% by weight of the composition.

26

37. A wound dressing composition according to any one of Claims 23-36 and including also a preservative.

38. A wound dressing composition according to any one of Claims 23-27, having a pH in the range 4 to 7.5.

39. A wound dressing composition according to Claim 38, having a pH in the range 4.5 to 6.5.

40. A wound dressing composition according to any one of Claims 23-39 and including also one or more biologically and/or pharmaceutically active components.

41. A sterile wound dressing composition according to any one of Claims 23-40.

42. A sterile wound dressing composition according to Claim 41 in a container for dispensing onto a wound site.